# EUROPEAN PATENT SPECIFICATION

(11) **EP 3 359 152 B1**
(45) Date of publication and mention of the grant of the patent: **27.05.2020**
(21) Application number: 16795263.9
(22) Date of filing: 04.11.2016
(51) Int. Cl.: A61K 31/44, A61K 31/00, A61K 45/06, A61P 3/06, A61P 9/10, A61P 9/12

(54) **USE OF PDE4 INHIBITORS FOR THE PROPHYLAXIS AND/OR THERAPY OF DYSLIPOPROTEINAEMIA AND RELATED DISORDERS**
VERWENDUNG VON PDE4 HEMMERN ZUR PROPHYLAXE UND/ODER THERAPIE VON DYSLIPOPROTEINAEMIE UND VERWANDTEN ERKRANKUNGEN
UTILISATION D'INHIBITEURS DE PDE4 POUR LA PROPHYLAXIE ET/OU LA THÉRAPIE DE LA DYSLIPOPROTEINÉMIE ET DES TROUBLES APPARENTÉS

(30) Priority: 09.11.2015 EP 15193646
(43) Date of publication of application: 15.08.2018
(73) Proprietor: Albert-Ludwigs-Universität Freiburg, 79098 Freiburg (DE)
(72) Inventor: AHLGRIM, Christoph, 79102 Freiburg (DE); BAUMSTARK, Manfred, 79111 Freiburg (DE); IDZKO, Marco, 79104 Freiburg (DE)
(74) Representative: Prüfer & Partner mbB Patentanwälte · Rechtsanwälte
(86) International application number: PCT/EP2016/076666
(87) International publication number: WO 2017/080919

(56) References cited:
- WO-A1-2004/105751
- WO-A1-2006/094933
- WO-A1-2011/019399
- WO-A2-2008/157537
- DE-A1- 10 156 230
- US-A1- 2012 046 237
- US-A1- 2015 018 292
- WONG H Y C (REPRINT) ET AL: "CHANGES IN HDL AND LDL LIPOPROTEINS BY DIAZEPAM IN THE REGRESSION OF EXPERIMENTAL ATHEROSCLEROSIS", ARTERIOSCLEROSIS, THE ASSOCIATION, DALLAS,TX, US, vol. 1, no. 5, 1 September 1981 (1981-09-01), page 389a, XP008179733, ISSN: 0276-5047
- HANY M. EL-BASSOSSY ET AL: "Chrysin and Luteolin Attenuate Diabetes-Induced Impairment in Endothelial-Dependent Relaxation: Effect on Lipid Profile, AGEs and NO Generation", PHYTOTHERAPY RESEARCH., vol. 27, no. 11, 7 January 2013 (2013-01-07), pages 1678-1684, XP055262509, GB ISSN: 0951-418X, DOI: 10.1002/ptr.4917
- MICHAEL LEHRKE ET AL: "PDE4 inhibition reduces neointima formation and inhibits VCAM-1 expression and histone methylation in an Epac-dependent manner", JOURNAL OF MOLECULAR AND CELLULAR CARDIOLOGY., vol. 81, 1 April 2015 (2015-04-01), pages 23-33, XP055262520, GB ISSN: 0022-2828, DOI: 10.1016/j.yjmcc.2015.01.015
- M. Kamper ET AL: "PP-351 Phosphodiesterase (PDE) IV inhibitor rolipram (ROL) diminishes endothelial dysfunction in experimental diabetes", FEBS Journal, 1 June 2006 (2006-06-01), pages 171-171, XP055263626, Retrieved from the Internet: URL:https://s3.amazonaws.com/objects.readc ube.com/articles/downloaded/wiley/c312dff7 590be6840e879cc4a638a09d605b6dd3333649951c b0bfd333ff6ec2.pdf?AWSAccessKeyId=AKIAIJZY FKH6APDFT3HA&Expires=1460160000&Signature= hyywWgRu8jsr0ValI4EexoBq0uA%3D&response-co ntent-type=application%2Fpdf [retrieved on 2016-04-07]
- GUALTIEROTTI ROBERTA ET AL: "Efficacy and Metabolic Effect on Serum Lipids of Apremilast in Psoriatic Arthritis: A Case Report.", JOURNAL OF CLINICAL MEDICINE 22 MAR 2019, vol. 8, no. 3, 22 March 2019 (2019-03-22), ISSN: 2077-0383
- XIANDONG LI ET AL.: "Animal models for the atherosclerosis research: a review", PROTEIN CELL, vol. 2, no. 3, 2011, pages 189-201,
- F. MÜLLERSHAUSEN: "Negatives "Feed-back" innerhalb der NO/cGMP-Signalkaskade", BIOSPEKTRUM, vol. 6, no. 03, 2003, pages 387-690,
- Anonymous: "Phosphodiesterase 4 Inhibitors", DrugBank, 27 August 2019 (2019-08-27), pages 1-2, XP055615601, Retrieved from the Internet: URL:https://www.drugbank.ca/categories/DBC AT001410 [retrieved on 2019-08-27]
- J. Lötvall, B. Lundbäck: "Phosphodiesterase inhibitors in obstructive lung disease" In: J. Lötvall: "Advances in combination therapy for asthma and COPD", 2012, John Wiley & Sons Ltd pages 296-299,
- DATABASE MEDLINE [Online] US NATIONAL LIBRARY OF MEDICINE (NLM), BETHESDA, MD, US; December 2011 (2011-12), LAHU GEZIM ET AL: "Pharmacokinetic evaluation of roflumilast.", Database accession no. NLM22059647 & LAHU GEZIM ET AL: "Pharmacokinetic evaluation of roflumilast.", EXPERT OPINION ON DRUG METABOLISM & TOXICOLOGY DEC 2011, vol. 7, no. 12, December 2011 (2011-12), pages 1577-1591, ISSN: 1744-7607
- Product monograph for Eucrisa (Crisaborole)

## Description

### TECHNICAL FIELD

The present invention relates to a new medication for the treatment (prophylaxis and/or therapy) of dyslipoproteinaemia, also called dyslipidemia, as well as of diseases or medical complications being related thereto, such as treatment of a lipoprotein imbalance present in the same serum of humans, more specifically an abnormally high low density lipoprotein (LDL) fraction, as defined in the claims.

### BACKGROUND ART

Dyslipoproteinaemia is a general diseases indication characterized by abnormal changes in lipoprotein transport, lipid metabolism, and lipoprotein concentrations in serum. Depending on the respective aetiology, pathologic conditions are generally classified into primary dyslipoproteinaemia as a result of genetic determinations, and secondary dyslipoproteinaemiasas a result and consequence of various basic originating diseases, such as adipositas, diabetes mellitus, alcoholism, liver diseases, or as a result of prior medications such as corticosteroids, diuretics, beta-blocker, etc.

According to the present invention, dyslipoproteinaemia also means abnormal cholesterol and/or triglyceride levels in the blood, notably human blood and serum, in particular hypercholesterinaemia, hypertriglyceridemia, or imbalance of lipoprotein profile and concentrations in the blood, especially human blood or serum. Notably, increased levels of cholesterol, triglycerides and/or lipoproteins, in particular the low density lipoproteins (LDL) are typically correlated with a risk of cardiovascular diseases.

Conventionally, the management of dyslipoproteinaemia/lipidaemia, or abnormal or imbalanced lipid/lipoprotein levels are commonly based on treatments with statins (HMG-CoA reductase inhibitors), or in the case of hypertriglyceridemia treatment with fibrates (peroxisome proliferator-activated receptor-α agonists). Statins are optionally combined with ezetimib, niacin, and bile acid sequestrants. Before medication, dietary modification or dietary supplementation, bodyweight reduction, and treatment of the basic originating disease in the event of secondary hyperlipoproteinaemia are usually the first approaches.

WO2006/094933A1 discloses e.g. roflumilast for use in the treatment of diabetes mellitus type 2, diabetes mellitus type 1 and disorders, which are related to diabetes mellitus.

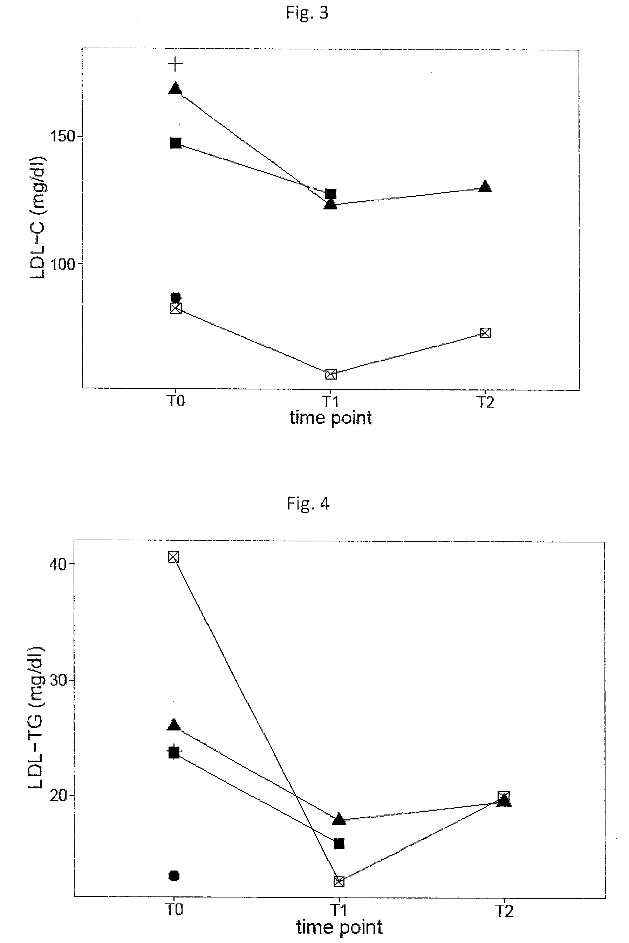

WO2011/019399A1 describes the use of PDE inhibitors in general, and in particular of PDE5 inhibitors to prevent, reduce or reverse the deleterious effects on blood vessels or to stimulate and enhance the growth of blood vessels. The examples show effects on eNOS protein levels (e.g. Fig. 3C) and angiogenesis (e.g. Fig. 6B) as direct effects on blood vessels.

Wong et al., Arteriosclerosis Council Abstracts, Vol. 1, No. 5, 1 September 1981, p. 389a indicate changes in HDL and LDL lipoproteins by diazepam (Valium) in the regression of experimental atherosclerosis in roosters.

El-Bassossy et al., Phytotherapy Research, Vol. 27, No. 11, pp. 1678-1684 (2013) reports that luteolin, a plant flavonoid compound, was tested to protect against vascular effects of diabetes in rats. US2015/018292A1 describes various *Ribes* alkaloid compositions (fractions) and their effect on different enzymes, e.g. IKK-β, PDE4 and PDE5 and the mitochondria. Example 9 shows the use of two different *Ribes* alkaloid compositions RAP2 and RAP4 and their effect on pigs.

### SUMMARY OF THE INVENTION

There is a need, and hence it is an object, to provide, especially by broadening and improving, the therapeutic possibilities of dyslipoproteinaemia and related disorders, including in particular pathologic and risk conditions associated with dyslipoproteinaemia and lipoprotein imbalance.

This object is solved by the subject-matter as defined in claim 1. Preferred embodiments are defined in subclaims. Any references in the description to methods of treatment refer to the compounds, pharmaceutical compositions, and medicaments of the present invention for use in a method for treatment of the human or animal body.

With the present invention, a completely novel therapeutic concept is established, offering broader and improved therapeutic possibilities for the treatment (prophylaxis and/or therapy) of dyslipoproteinaemia, and of diseases correlated or associated with dyslipoproteinaemia or lipoprotein imbalance, in particular imbalanced lipoprotein profiles and specifically abnormal high lipoprotein levels, especially increased LDL. Accordingly, the present invention establishes a new therapeutic approach to reduce the risk of cardiovascular diseases, in particular arteriosclerosis, myocardial infarction, stroke, peripheral artery disease, and vascular stenosis or restenosis, when respectively correlated or associated with dyslipoproteinaemia or lipoprotein imbalance.

As a basis for the afore-mentioned new therapeutic concepts, it was surprisingly found that inhibitors of phosphodiesterase 4 (PDE 4) are capable of decreasing lipoprotein levels present in blood, notably in human blood and serum. More surprisingly, and particularly valuable for the above specified therapeutic applications, critical lipoprotein fractions and their lipid components cholesterol and/or triglyceride, and in particular LDL and LDL-cholesterol, have been found to be selectively and remarkably decreased upon administration of PDE4 inhibitors.

### BRIEF DESCRIPTION OF THE DRAWINGS

Fig. 1 shows changes in the blood serum levels (in absolute amounts mg/dl) of cholesterol depending on specific lipoprotein fractions upon administration of a PDE4 inhibitor, respectively after 4 weeks (T1) and after 8 weeks (T2), compared to the initial values at start (T0);
Fig. 2 shows changes in the blood serum levels (in absolute amounts mg/dl) of triglycerides depending on specific lipoprotein fractions upon administration of a PDE4 inhibitor, respectively after 4 weeks (T1) and after 8 weeks (T2), compared to the initial values at start (T0);
Figs. 3 and 4 respectively shows the course of individual changes of LDL-C and LDL-TG from start (T0) until T2 in the blood serum levels (in mg/dl) of LDL-C (Fig. 3) and LDL-TG (Fig. 4).

### DESCRIPTION OF PREFERRED EMBODIMENTS

As known, phosphodiesterase 4 (PDE 4) is a member of a large, divergent family of phosphodiesterase (PDE) enzymes that catalyse the catabolism of cAMP and cGMP to AMP and GMP, respectively. PDE4 itself encompasses four types, PDE4A, PDE4B, PDE4C and PDE4D, respectively sub-divided into various isoforms. According to the present invention, PDE 4-specific inhibitors are used. All PDE4 types and isoforms are meant, and inhibitors specific for each PDE4 type can be used according to the present invention. Literature and reviews about PDE4, its subtype isoforms and the inhibitory class effect of PDE4 inhibitors can be found, for example, in H. Wang et al. in Biochem.J. 408, 193-201 (2007); K.F. Rabe in Br J Pharmacol. 163(1), 53-67 (2011); Hatzelmann and Schudt in JPET 297, 267-279 (2001).

The new therapeutic effect found for PDE4 inhibitors according to the present invention credibly can be achieved as the function of the class of PDE4 inhibitors.

Prior therapeutic uses of PDE4 inhibitors are known and widespread. However, since the PDE4 family of enzymes are most prevalent in immune cells and cells in the central nervous system, their therapeutic utility hitherto has been limited essentially in diseases and pathologic disorders associated with inflammation and nervous system disorders, based on neuroprotective and anti-inflammatory effects. Consequently, PDE4 inhibitors have been investigated as treatments for a diverse group of different diseases, including central nervous system disorders such as major depressive disorder (clinical depression), anxiety disorders, schizophrenia, Parkinson's disease, Alzheimer's disease, multiple sclerosis, attention deficit-hyperactivity disorder, Huntington's disease, autism and inflammatory conditions such as chronic obstructive pulmonary disease (COPD), asthma and rheumatoid arthritis.

PDE4 inhibitors have also been described in relationship with cardiac and cardiovascular diseases, however only within the classical PDE4-correlated and -associated inflammatory-related conditions and circumstances. Examples of such conventional, inflammation-related therapies of PDE4 inhibitors are e.g. WO2009/067600A and WO2004/050624A. In the context of such classical therapeutic approach, WO2004/105751A further describes the treatment of cardiac hypertrophy by using a PDF4 inhibitor.

PDE4 inhibitors inhibit the degradation of cAMP into AMP. Since cAMP is a second messenger important in many biological processes and acts by intracellular signal transduction in many different organisms, conveying cAMP-dependent pathways, typically through activation of protein kinases, and are thereby involved in several biochemical processes, including the regulation of glycogen, sugar, and lipid metabolism (e.g. activation of lipases). Among a vast number of different reports and investigations about possible metabolic effects of PDE 4 inhibitors, Lynn and Bornfeldt in Biochemical and Biophysical Research Communications 290, 663-669 (2002) report about PDE4 inhibitors to promote the expression of ATP cassette binding protein 1 (ABCA1) and cholesterol efflux from THP-1 cells and macrophages.

By the newly found capability of PDE4 inhibitors to beneficially influence lipoprotein profiles and in particular by their capability to counter-act lipoprotein imbalance in blood and serum of humans, the present invention now provides the possibility of treating endothelial dysfunction and cardiovascular diseases when correspondingly correlated or associated with dyslipoproteinaemia or lipoprotein imbalance, in particular abnormally high low density lipoprotein (LDL) in blood or serum. Accordingly, particularly useful treatments include for example arteriosclerosis, myocardial infarction, stroke, peripheral artery diseases, vascular stenosis, respectively independent from, or not correlated with or associated with inflammatory diseases or conditions such as inflammatory-associated cardiovascular pathology and cardiac hypertrophy. For distinction between dyslipoproteinaemia or lipoprotein imbalance associated circumstances and inflammatory-associated circumstances (especially in terms of cardiovascular events), reference can be made to März et al., Circulation 110:3068-3074 (2004), demonstrating the different underlying biochemical mechanisms and situations with respect to cardiovascular risks depending on whether lipoprotein metabolism or inflammatory processes are involved. That is, distinct from such inflammatory-originating pathways and associated diseases or conditions and inflammatory-associated cardiovascular pathology, the present invention beneficially allows prophylactic and/or therapeutic treatments of endothelial dysfunction and cardiovascular diseases characterized by different clinical settings, especially in circumstances of abnormal high LDL levels and especially LDL-cholesterol and LDL-triglyceride levels.

Accordingly, the phosphodiesterase 4 inhibitors for use according to the present invention are particularly useful in the prophylaxis and/or therapy for reducing cardiovascular risk, and for reducing the risk of re-infarction. As standard references for such reduced risks, published practical guidelines of the American College of Cardiology (ACC) and the American Heart Association (AHA) on levels of blood cholesterol and in particular of LDL-C can be referred to, see e.g. K.K. Ray et al. in European Heart Journal advance access published March 17, 2014: "The ACC/AHA 2013 guideline on the treatment of blood cholesterol to reduce atherosclerotic cardiovascular disease risk in adults: the good the bad and the uncertain: a comparison with ESC/EAS guidelines for the management of dyslipidaemias 2011*".* Regarding blood TG and LDL-TG, reference can be made to März et al., *supra,* in Circulation 110:3068-3074 (2004).
Lipoproteins, cholesterol and triglycerides are usually analysed from serum samples but can also be assessed from plasma. Normal values for TG are below 150 mg/dl. Normal values for cholesterol are below 200mg/dl. In current guidelines, total cholesterol is seen as a risk factor for cardiovascular events. Indications for treatment of hypercholesterinaemia may depend on LDL-C value and other cardiovascular risk factors.

Current guidelines advice that therapy may already be initiated in patients with LDL-C values as low as 70 mg/dl when they have a high cardiovascular risk. On the other side of the spectrum patients with a low cardiovascular risk are considered for treatment when LDL-C levels exceed 190 mg/dl.
Target values for LDL-C in lipid lowering therapy are between 70mg/dl and 115 mg/dl depending on the cardiovascular risk.
Regarding LDL-TG as risk factor of cardiovascular diseases, März et al. (*supra*) discussed increased Odd's ratios for cardiovascular disease in depending on the quantile of LDL-TG distribution pointing towards a continuous association between LDL-TG levels and cardiovascular risk.
Furthermore, and based on the finding of a new effectivity of PDE4 inhibitors, the prophylactic and/or therapeutic treatment is associated with a decrease in LDL-associated cholesterol (LDL-C) and/or is associated with a decrease of a ratio of low density lipoprotein to high density lipoprotein (i.e. decreasing the LDL/HDL-ratio in the blood, notably the serum of humans).
Similarly and again based on the findings of the present invention, the treatment of prophylaxis and/or therapy is associated with a decrease in low density lipoprotein-triglycerides (LDL-TG).
It is preferred that during the treatment (prophylaxis and/or therapy) the term "said decrease" means that the respective decrease is at least 10%, more preferably at least 20%. Our observations indicate that PDE4 inhibitors lower LDL-C by up to 45mg/dl (30%). The effect on LDL-TG is more pronounced as LDL-TG was reduced by up to 29 mg/dl (60%) in our observations. Optimisations may achieve more.

Active compounds from the class of PDE4 specific inhibitors (i.e. having the function of inhibiting phosphodiesterase 4 specifically) are used according to the present invention. These include, without being limited to, specific PDE4 inhibitors selected from the group consisting of: 3-cyclopropylmethoxy-4-difluoromethoxy-N- (3, 5-dichloropyrid-4-yl)-benzamide (Roflumilast) as well as Roflumilast-N-Oxide, Apremilast, Piclamilast, cis-4-cyano-4- [3-cyclopentyloxy-4-methoxyphenyl] cyclohexane-1-carboxylic acid (Cilomilast), AN2728 (crisaborole), Oglemilast, Revamilast, E6005, Ronomilast, Rolipram; and pharmaceutically acceptable salts of the aforementioned compounds. Roflumilast is preferred as it has the advantage of being approved by health authorities.
Examples of the above listed PDE4 inhibitors and their synthesis are for example described in WO2004/103407A, WO02/064584, WO95/01338, W092/12961, W093/19749, WO95/00516, WO96/11690, WO98/09946, WO98/09946, WO97/23457, WO00/26208, EP0435811, EP0731099, and EP0389282, and in representative literature references including e.g. those of Wang et al. (2007) and Rabe (2011), *supra.*

The present invention further provides a pharmaceutical composition for the above-described therapeutic uses, wherein in the composition the PDE4 inhibitor is contained together with a pharmaceutically acceptable excipient, carrier, diluent, and/or additive (inactive ingredients). The composition can be formulated in a suitable administration and dosage form, for example as a solid, a semi-solid, gelatinous, a liquid, a suspension, a powder, a tablet, a pill, a capsule, a sustain-released dosage form, and the like. Furthermore, the administration and dosage form may for example be oral, parenteral, transdermal, nasal, for injection or for inhalation. Examples of suitable inactive ingredients and suitable administration forms are given in the above mentioned PDE 4 inhibitor related WO and EP publications.

The PDE4 inhibitor, either alone or when present in the afore-mentioned pharmaceutical composition, is used in a suitable amount effective for the prophylaxis and/or therapy of the afore-mentioned diseases or diseases conditions. For example, a suitable amount or dose of a PDE4 inhibitor is at least 0.0001 mg/kg, for example in a range of from 0.0001 to 1000 mg/kg, preferably from 0.01 to 100 mg/kg and particularly from 0.1 to 20 mg/kg by weight of the treated patient. Doses can be administered singly or repeatedly, locally or systemically, once or several times daily or weekly, or - especially of depot or slow-release formulations - optionally also at a lower frequency. It may also be useful to apply time-dependent dosage regimen, starting with a first unit dose, for example a daily dose of 50 mg-100 mg, preferably 250 mg-750 mg, and after some weeks of for example a second unit dose different from the first unit dose, for example being twice as high as the first unit dose. The dose and the time-dependent dosage regimen can be followed and controlled by, and associated with the measurement of, lipoprotein profiles and lipoprotein levels, in particular LDL and HDL levels, respectively their fractions, in particular LDL-cholesterol and LDL-triglyceride, respectively alone or in combination.

In a further aspect of the present invention the PDE4 inhibitor is combined with another compound active against, or reducing the risk of, dyslipidaemia, lipoprotein imbalance, endothelial dysfunction, cardiovascular diseases, arteriosclerosis, hypertension and stroke. Examples of such other active compounds can be selected, for instance, from the group of statins. Since, as described above, the mechanism and approach of treating endothelial dysfunction and cardiovascular diseases according to the present invention of using PDE4 inhibitors - by being correlated or associated with dyslipoproteinaemia, lipoprotein imbalance and in particular hypercholesterinaemia or hypertriglyceridemia - is distinct from other risk factors and other aetiologies and causes of cardiovascular disease and endothelial dysfunction, in particular distinct from inflammatory-associated conditions, it is evident that combinatory effects can be achieved to thereby provide overall improved therapies of endothelial dysfunctions and cardiovascular diseases. Accordingly, the present invention for example further provides a useful combined medication, wherein a phosphodiesterase 4 inhibitor and a HMGCoA inhibitor, preferably selected from the group of statins, are combined for use in a treatment of cardiovascular diseases, arteriosclerosis, hypertension, dyslipoproteinaemia, or for reducing the risk of any one the these diseases. The combination can be embodied in separate or in common dosage forms, and can be administered concurrently or temporarily shifted according to a desirable dose and time regimen.

The present invention is further described by reference to non-limiting and representative examples in the following.

### Examples

In a clinical trial, five patients were treated by daily doses of a PDE4 inhibitor. As an exemplifying and representative compound, roflumilast was used as the PDE4-specific inhibitor. Lipoprotein profiles were measured at start of the therapy (time T0), four weeks after start (T1) and eight weeks after start (T2). Treatment was further monitored thereafter. In an embodiment, a first unit dose (500 mg) was administered orally every second day for a period of four weeks, and subsequently the dose was increased to a unit of 500 mg per day until a time of 8 weeks.

For analysing and measuring the lipoprotein profiles and thus the relative concentrations and levels of lipoprotein fractions and the cholesterol and triglyceride amounts per lipoprotein, 10 ml venous blood was drawn at the indicated time points. Lipoprotein profiles and fractions were determined using density separation by means of ultra-centrifugation and subsequent determination of the lipoprotein components (Baumstark et al., Biochem. Biophys. Acta - Protein Structure and Molecular Enzymology 1037, 48-57 (1990); Frey et al., Eur. J. Appl. Physiol. 60, 441-444 (1990)). The components and parameters determined include: cholesterol and triglyceride proportions of HDL and LDL, and of sub-fractions LDL-1 to LDL-6, and HDL-2a-2b and -3. Statistical analysis of the results was carried out by SPSS 23.0.

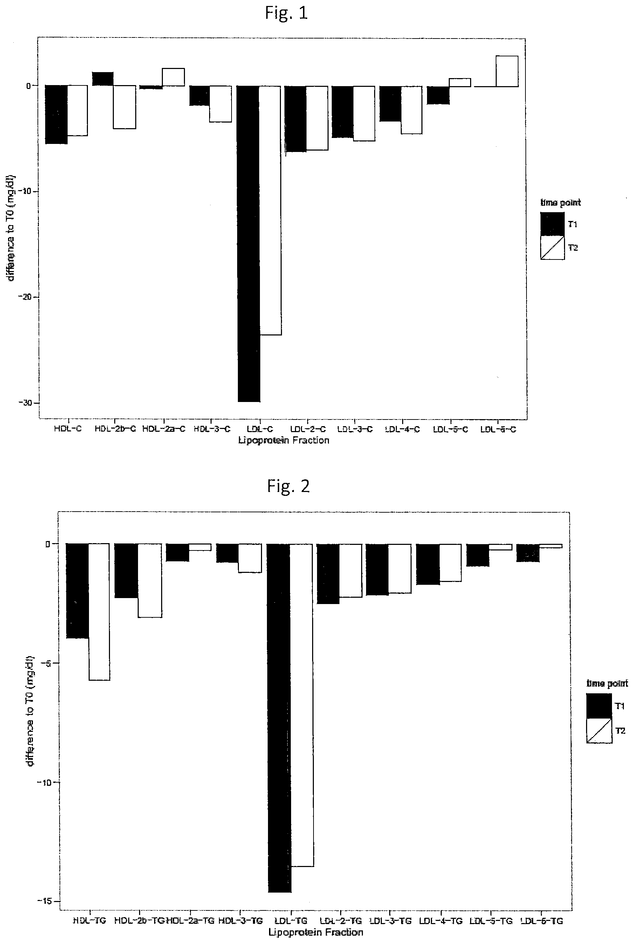

The changes, in absolute amounts of mg/dl, of the cholesterol proportions of the lipoprotein fractions at time T1 and T2 compared to T0 are shown in Fig. 1.

Furthermore, the absolute changes, in mg/dl, of the triglyceride proportions of the lipoprotein fractions at time T1 and T2 in comparison with T0 is shown in Fig. 2.

The individual changes of LDL-T and LDL-TG in the patient's blood are shown in Figs. 4 and 5.

As shown in the Figures, a substantial reduction of certain lipoprotein sub-fractions, in particular a remarkable reduction of LDL-C by approximately 30 mg/dl was observed. LDL-TG was also substantially reduced. The reduction of LDL-C and LDL-TG are maintained over longer time periods, notably after 12 weeks (not shown).

Accordingly, it is demonstrated that, independent from effects on systemic inflammation, the PDE4-specific inhibitor can effectively improve therapeutic concepts, such as reduction of cardiovascular risks, by a reduction of independent cardiovascular risk factors, in particular by reduction by LDL-C. Further independently from LDL-C, also a reduction of LDL-TG is possible, as further independent beneficial influence on the cardiovascular risk and as indications of further therapeutic beneficial situations. Slight reductions of HDL-C and HDL-TG were also observed.

Accordingly, it has been shown that PDE4-specific inhibitors have a beneficial influence on the systemic lipoprotein profile in human blood and serum, in particular by improving the LDL to HDL ratio, and in particular by remarkably reducing the LDL-C and HDL-C, but independently also the LDL-TG. Accordingly, the use of PDE4-specific inhibitors can help to reduce cardiovascular risks, and furthermore can establish effective treatments in systemic lipoprotein-related disorders, such as dyslipoproteinaemia, and diseases correlated/associated with dyslipoproteinaemia, lipoprotein imbalance or related disorders.

## Claims

1. Phosphodiesterase 4 (PDE 4)-specific inhibitor for use in the prophylactic or therapeutic treatment of dyslipoproteinaemia, wherein the PDE 4-specific inhibitor decreases low density lipoprotein-associated cholesterol (LDL-C) and/or low density lipoprotein-associated triglyceride (LDL-TG); and/or the ratio of low density lipoprotein (LDL) to high density lipoprotein (HDL), respectively, in human blood or serum.

2. Phosphodiesterase 4 -specific inhibitor for use according to claim 1, wherein the dyslipoproteinaemia is associated with any of the following diseases:
endothelial dysfunction, cardiovascular diseases, in particular arteriosclerosis, myocardial infarction, stroke, peripheral artery disease, and vascular stenosis or restenosis.

3. Phosphodiesterase 4-specific inhibitor for use according to claim 1 or 2, in the prophylaxis and/or therapy for reducing cardiovascular risk, or for reducing the risk of re-infarction.

4. Phosphodiesterase 4-specific inhibitor for use according to any one of claims 1 to 3, wherein the prophylaxis and/or therapy is not correlated or associated with inflammatory-originating and -associated diseases or conditions, in particular not correlated or associated with inflammatory-associated cardiovascular pathology.

5. Phosphodiesterase 4-specific inhibitor for use according to any one of claims 1 to 4, wherein the PDE4-specific-inhibitor is selected from the group consisting of 3-cyclopropylmethoxy-4-difluoromethoxy-N- (3, 5-dichloropyrid-4-yl)-benzamide (Roflumilast) as well as Roflumilast-N-Oxide, Apremilast, Piclamilast, cis-4-cyano-4- [3-cyclopentyloxy-4-methoxyphenyl] cyclohexane-1-carboxylic acid (Cilomilast), AN2728 (crisaborole), Oglemilast, Revamilast, E6005, Ronomilast, Rolipram; and the pharmaceutically acceptable salts thereof.

6. Phosphodiesterase 4-specific inhibitor for use according to claim 4, wherein during the prophylaxis and/or therapy said decrease is at least 10%.

7. Phosphodiesterase 4-specific inhibitor for use according to claim 4, wherein during the prophylaxis and/or therapy said decrease is at least 20%.

8. Phosphodiesterase 4-specific inhibitor for use according to any one of claims 1 to 7, wherein the single dose of the PDE4-inhibitor in the oral administration is 0.01 to 10 mg PDE 4-inhibitor per kg body weight.

9. Phosphodiesterase 4-specific inhibitor for use according to any one of claims 1 to 7, wherein the single dose of the PDE4-inhibitor in the oral administration is 0.1 to 1,5 mg PDE 4-inhibitor per kg body weight.

10. Phosphodiesterase 4-specific inhibitor for use according to any one of claims 1 to 8 in combination with another compound active against cardiovascular diseases, arteriosclerosis, hypertension, dyslipoproteinaemia.

11. A phosphodiesterase 4-specific inhibitor for use according to any one of claims 1 to 9 in combination with an HMGCoA inhibitor, wherein the use is for a treatment of cardiovascular diseases, arteriosclerosis, hypertension, dyslipoproteinaemia, or for reducing the risk of any one the these diseases.

12. Phosphodiesterase 4-specific inhibitor for use according to any one of claims 1 to 10, wherein the PDE4 specific-inhibitor is contained in a pharmaceutical composition with a pharmaceutically acceptable excipient, carrier, diluent or additive.

## Patentansprüche

1. Phosphodiesterase 4 (PDE 4)-spezifischer Inhibitor zur Verwendung bei der prophylaktischen oder therapeutischen Behandlung von Dyslipoproteinämie, wobei der PDE 4-spezifische Inhibitor das Lipoprotein geringer Dichte-assoziierte Cholesterin ("low density lipoprotein"; LDL-C) und/oder das Lipoprotein geringer Dichte-assoziierte Triglycerid (LDL-TG) und/oder das Verhältnis von Lipoprotein geringer Dichte (LDL) zu Lipoprotein hoher Dichte ("high density lipoprotein"; HDL) im menschlichen Blut oder Serum senkt.

2. Phosphodiesterase 4 -spezifischer Inhibitor zur Verwendung nach Anspruch 1, wobei die Dyslipoproteinämie mit einer der folgenden Krankheiten assoziiert ist:
endotheliale Dysfunktion, kardiovaskuläre Erkrankungen, insbesondere Arteriosklerose, Myokardinfarkt, Schlaganfall, periphere Arterienerkrankung und Gefäßverengung oder Restenose.

3. Phosphodiesterase-4-spezifischer Inhibitor zur Verwendung nach Anspruch 1 oder 2, bei der Prophylaxe und/oder Therapie zur Verringerung des kardiovaskulären Risikos oder zur Verringerung des Risikos eines Re-Infarktes.

4. Phosphodiesterase-4-spezifischer Inhibitor zur Verwendung nach irgendeinem der Ansprüche 1 bis 3, wobei die Prophylaxe und/oder Therapie nicht korreliert mit oder assoziiert ist mit Krankheiten oder Zuständen, die von Entzündungen ausgehen und mit Entzündungen assoziiert sind, insbesondere nicht korreliert mit oder assoziiert ist mit entzündungsassoziierter kardiovaskulärer Pathologie.

5. Phosphodiesterase-4-spezifischer Inhibitor zur Verwendung nach irgendeinem der Ansprüche 1 bis 4, wobei der PDE4-spezifische Inhibitor aus der Gruppe ausgewählt ist bestehend aus 3-Cyclopropylmethoxy-4-difluormethoxy-N- (3, 5-Dichlorpyrid-4-yl)-benzamid (Roflumilast) sowie Roflumilast-N-Oxid, Apremilast, Piclamilast, cis-4-Cyano-4- [3-Cyclopentyloxy-4-methoxyphenyl] cyclohexan-1-carbonsäure (Cilomilast), AN2728 (Crisaborol), Oglemilast, Revamilast, E6005, Ronomilast, Rolipram; und die pharmazeutisch verträglichen Salze davon.

6. Phosphodiesterase-4-spezifischer Inhibitor zur Verwendung nach Anspruch 4, wobei während der Prophylaxe und/oder Therapie die Abnahme mindestens 10% beträgt.

7. Phosphodiesterase-4-spezifischer Inhibitor zur Verwendung nach Anspruch 4, wobei während der Prophylaxe und/oder Therapie die Abnahme mindestens 20% beträgt.

8. Phosphodiesterase-4-spezifischer Inhibitor zur Verwendung nach irgendeinem der Ansprüche 1 bis 7, wobei die Einzeldosis des PDE4-Inhibitors bei der oralen Verabreichung 0,01 bis 10 mg PDE4-Inhibitor pro kg Körpergewicht beträgt.

9. Phosphodiesterase-4-spezifischer Inhibitor zur Verwendung nach irgendeinem der Ansprüche 1 bis 7, wobei die Einzeldosis des PDE4-Inhibitors bei der oralen Verabreichung 0,1 bis 1,5 mg PDE4-Inhibitor pro kg Körpergewicht beträgt.

10. Phosphodiesterase-4-spezifischer Inhibitor zur Verwendung nach irgendeinem der Ansprüche 1 bis 8 in Kombination mit einer anderen Verbindung, die gegen Herz-Kreislauf-Erkrankungen, Arteriosklerose, Bluthochdruck, Dyslipoproteinämie wirksam ist.

11. Phosphodiesterase-4-spezifischer Inhibitor zur Verwendung nach irgendeinem der Ansprüche 1 bis 9 in Kombination mit einem HMGCoA-Inhibitor, wobei die Verwendung für eine Behandlung von kardiovaskulären Erkrankungen, Arteriosklerose, Bluthochdruck, Dyslipoproteinämie oder zur Verringerung des Risikos einer dieser Erkrankungen erfolgt.

12. Phosphodiesterase-4-spezifischer Inhibitor zur Verwendung nach irgendeinem der Ansprüche 1 bis 10, wobei der PDE4-spezifische Inhibitor in einer pharmazeutischen Zusammensetzung mit einem pharmazeutisch annehmbaren Hilfsstoff, Träger, Verdünnungsmittel oder Additiv enthalten ist.

## Revendications

1. Inhibiteur de phosphodiestérase 4 (PDE 4) pour utilisation dans le traitement prophylactique ou thérapeutique d'une dyslipoprotéinémie, dans lequel l'inhibiteur de PDE 4 diminue le cholestérol lié aux lipoprotéines de basse densité (LDL-C) et/ou les triglycérides liées aux lipoprotéines de basse densité (LDL-TG) ; et/ou le taux de lipoprotéines de basse densité (LDL) par rapport aux lipoprotéines de haute densité (HDL), respectivement dans du sang humain ou du sérum.

2. Inhibiteur de phosphodiestérase 4 pour utilisation selon la revendication 1, dans lequel la dyslipoprotéinémie est associée à l'une des maladies suivantes :
un dysfonctionnement endothélial, des maladies cardiovasculaires, en particulier l'artériosclérose, un infarctus du myocarde, un accident vasculaire cérébral, une maladie artérielle périphérique, et une sténose ou resténose vasculaire.

3. Inhibiteur de phosphodiestérase 4 pour utilisation selon la revendication 1 ou 2, dans la prophylaxie et/ou le traitement destiné à réduire le risque cardiovasculaire, ou à réduire le risque de récidive d'infarctus.

4. Inhibiteur de phosphodiestérase 4 pour utilisation selon l'une quelconque des revendications 1 à 3, dans lequel la prophylaxie et/ou le traitement n'est pas corrélé ou associé à des maladies ou affections d'origine inflammatoire ou associées à un état inflammatoire, en particulier non corrélé ou associé à une pathologie cardiovasculaire associée à un état inflammatoire.

5. Inhibiteur de phosphodiestérase 4 pour utilisation selon l'une quelconque des revendications 1 à 4, dans lequel l'inhibiteur de PDE4 est choisi dans le groupe consistant en 3-cyclopropylméthoxy-4-difluorométhoxy-N-(3,5-dichloropyrid-4-yl)-benzamide (Roflumilast) ainsi que Roflumilast-N-Oxyde, Aprémilast, Piclamilast, acide cis-4-cyano-4-[3-cyclopentyloxy-4-méthoxyphényl]cyclohexane-1-carboxylique (Cilomilast), AN2728 (crisaborole), Oglémilast, Révamilast, E6005, Ronomilast, Rolipram ; et leurs sels pharmaceutiquement acceptables.

6. Inhibiteur de phosphodiestérase 4 pour utilisation selon la revendication 4, dans lequel durant la prophylaxie et/ou le traitement ladite diminution est d'au moins 10 %.

7. Inhibiteur de phosphodiestérase 4 pour utilisation selon la revendication 4, dans lequel durant la prophylaxie et/ou le traitement ladite diminution est d'au moins 20 %.

8. Inhibiteur de phosphodiestérase 4 pour utilisation selon l'une quelconque des revendications 1 à 7, dans lequel la dose unique d'inhibiteur de PDE4 par administration orale est de 0,01 à 10 mg d'inhibiteur de PDE 4 par kg de poids corporel.

9. Inhibiteur de phosphodiestérase 4 pour utilisation selon l'une quelconque des revendications 1 à 7, dans lequel la dose unique d'inhibiteur de PDE4 par administration orale est de 0,1 à 1,5 mg d'inhibiteur de PDE 4 par kg de poids corporel.

10. Inhibiteur de phosphodiestérase 4 pour utilisation selon l'une quelconque des revendications 1 à 8 en combinaison avec un autre composé actif contre des maladies cardiovasculaires, une artériosclérose, une hypertension, une dyslipoprotéinémie.

11. Inhibiteur de phosphodiestérase 4 pour utilisation selon l'une quelconque des revendications 1 à 9 en combinaison avec un inhibiteur de HMGCoA, dans lequel l'utilisation est destinée au traitement de maladies cardiovasculaires, d'une artériosclérose, d'une hypertension, d'une dyslipoprotéinémie, ou à réduire le risque de l'une quelconque de ces maladies.

12. Inhibiteur de phosphodiestérase 4 pour utilisation selon l'une quelconque des revendications 1 à 10, dans lequel l'inhibiteur de PDE 4 est contenu dans une composition pharmaceutique avec un excipient, porteur, diluent ou additif pharmaceutiquement acceptable.
